# EUROPEAN PATENT APPLICATION

(11) **EP 2 071 035 A1**
(43) Date of publication of application: **17.06.2009**
(21) Application number: 08171443.8
(22) Date of filing: 12.12.2008
(51) Int. Cl.: C12Q 1/68

(54) **Facilitator and method for amplification**

(30) Priority: 13.12.2007 EP 07123136
(71) Applicant: Epigenomics AG, 10178 Berlin (DE)
(72) Inventor: Schuster, Matthias, Dr., 13156 Berlin (DE)

(57) **Abstract**

Subject matter of the invention is a method for amplification of nucleic acids, wherein a successful binding of a primer and a facilitator onto the template is required for amplification. The invention further comprises the inventive facilitator, inventive kits comprising a facilitator and the use of said inventive method, facilitator or kit.

## Description

### FIELD OF THE INVENTION

The invention relates generally to novel and substantially improved components, methods and kits for amplification of nucleic acids.

### BACKGROUND OF ASPECTS OF THE INVENTION

Amplification methods and correspondent components are well known in the art. Well known methods are for example polymerase chain reaction (PCR), ligase chain reaction (LCR), isothermal amplification such as primer extension or strand displacement amplification (SDA), and RNA based amplification methods such as NASBA (nucleic acid sequence based amplification). Amplification reactions usually comprise a molecule that is elongated, one or more molecules by which it is elongated such as oligomers or monomers and one or more enzymes catalyzing the amplification reaction.

NASBA is an isothermal amplification method usually being carried out at 42°C. The enzymatic components are reverse transcriptase, RNAse H, and T7 RNA polymerase. Usually, the T7 promotor is part of a primer normally located on its 5' end. The reaction is started by the hybridization of the T7 promotor comprising primer onto the RNA template. Thereafter the RNA is reverse transcriptized to cDNA by means of the T7 RNA polymerase. The RNA of the resulting hybrid molecule is then digested by RNAse H. The complementary reverse strand is then generated by means of the second primer and the DNA dependent DNA polymerising activity of the reverse transcriptase. During this step also the T7 promotor is newly synthesized. In the next step the T7 polymerase binds to its promotor and generates about 100 new RNA strands per DNA strand. The self-sustained sequence replication (3SR) method is based on the same principle. Both methods can be easily adopted for the amplification of DNA.

SDA belongs also to the isothermal amplification methods. It is based on the fact that polymerases are able to start the synthesis of new strands at single strand breaks (nick sites) by displacing the old strand. Amplification is carried out by alternate incorporation of single strand breaks followed by strand displacement. The single strand breaks are introduced by a primer comprising the restriction site and by the use of 3 normal dNTPs and one thionucleotide. Through this a hybrid molecule is formed, wherein the sulfur comprising strand can not be digested by the restriction enzyme. Instead, only a single strand break is generated in the sulfur non-containing strand.

MDA (multiple displacement amplification) is a SDA method, wherein a varierty of random short primers are used. This leads to an amplification of the whole applied DNA i.e. to whole genome amplification.

LCR is an amplification method wherein two oligonucleotides or oligomers are hybridized next to each other on a template and are subsequently ligated by means of a heatstable ligase. The so formed product is then itself the template for two reverse complementary oligonucleotides or oligomers which after hybridization and ligation form the reverse complementary strand. In some variants LCR is combined with primer extension/PCR. Thereby the neighbouring oligonucleotides hybridize with a gap relative to each other. This gap is filled up by an extension of one of the oligonucleotides. The extended oligonucleotide immediately terminates at the end of the other oligonucleotide and is ligated to the said.

Primer extension is a linear amplification method. In a first step a primer is hybridized to a template. Subsequently, it is elongated by means of nucleotides and a polymerase. Well known primer extension methods are for example sequencing reactions and rolling circle amplification (RCA), in the later a single-stranded nucleic acids molecule is the template on to which the primer to be extended anneals.

The maybe best known amplification method is PCR. According to it, in a first step double stranded DNA is denatured. In the second step, a respective oligonucleotide capable of being elongated is hybridized to each individual strand. These oligonucleotides are also known as primers. In a third step the hybridized primers are elongated wherein double stranded DNA is generated. The elongation product of one primer comprises also the primer binding site of the respectively other primer. Thereafter the denaturing, primer annealing and elongation is repeated for several times in so-called cycles.

PCR has a wide applicability. It comprises amongst others the multiplication of a template, the generation of cDNA from RNA, and the detection and quantification of a molecule or sequence of interest. In case of quantification, the so-called real time PCR method is performed many times. Real time PCR is based thereon that the amount of amplificates is detected after each cycle of amplification. For this, intercalating dyes or labeled probes, or primers are used. A molecule or sequence of interest is quantified by comparing the amount of amplificate generated over time with the corresponding amount of amplificate of a reference. A person skilled in the art is aware of numereous methods for real time PCR analysis. Non-limiting examples are: TaqMan® detection technology; dual-probe technology (LightCycler^{™}), fluorescent amplification primers (Sunrise^{™} technology), Molecular Beacon Probes (Tyagi S., and Kramer F.R., Nature Biotechnology 14, 303-308, 1996), Scorpion primers (Whitcombe et al., Nature and Biotechnology, 17, 804-807, 1999), LNA (Locked Nucleid Acid) Double-Dye Oligonucleotide probes (Exiqon A/S) or catalytic nucleic acid activities (US 6,140,055, or EP07111614).

In general, it is possible to amplify specifically only one single site as well as several or multiple single sites by means of PCR. The later can be achieved by multiplexing single site amplification reactions. In addition, it is also possible to amplify multiple sites unspecifically up to a whole genome amplification by means of degenerate primers or by means of adaptors.

The specificity of a PCR reaction is essentially determined by the choice of the reaction conditions. Reaction conditions determine the stringency with which a primer hybridizes to its target region. In order to make an amplification reaction highly specific, reaction conditions are chosen that allow the primers just to bind to specific target sites. In order to make an amplification more unspecific, conditions are chosen so that the primer may bind to multiple sites.

Moreover, the specificity of a PCR reaction is also essentially determined by the sequence of the primers. In principle, three types of primers are known: (a) sequence specific primer, (b) degenerate primer, and (c) short random primer. Short random primers are mainly used for whole genome amplification (for example but not limited to MDA) as well as for the amplification of RNA. Degenerate primers are a mixtures of primers that differ in defined positions of their sequence. They are mainly used in case the sequence of a target of interest is not completely known, or in case targets vary in their sequence. An examplary method is AP-PCR (arbitrary primed PCR). Probably, the most used primers are sequence specific primers. In order to achieve the sequence specificity, several rules of thumb have to be considered, for example: (1) the primer length is mostly in the range of 17-28 nucleotides, (2) ratio between GC content versus AT content is balanced, (3) the primer's melting temperature should be in the range of 55-80°C, and (4) both primers have preferably the same or a similar melting temperature with the target.

In addition, the specificity of a PCR can be increased by means of so-called blockers. A blocker is a oligonucleotide or oligomer that has a not-extendable 3' terminal end. It is usually applied in amplification reactions wherein a variant of a sequence has to be amplified in the presence of another variant of the same sequence. It is chosen to be specific for the variant that is not of interest. A blocker has usually a hybridization site that overlaps at least partially with the hybridization site of one primer. Because of this, the amplification of the unwanted variant is repressed for the benefit of the variant of interest. Examples for the usage of blockers are the allel-specific amplification and detection of SNPs or the detection of methylation according to the HeavyMethyl method (US7,229,759; WO 02/072880). Like sequence specific primers, blockers are sequence specific. In order to achieve the sequence specificity, several rules of thumb have to be considered, for example: (1) the blocker length is mostly in the range of 17-35 nucleotides, (2) the blocker's melting temperature should be in the range of 55-80°C, and (3) the blocker has a higher melting temperature with the target as the melting temperature of the two used primers with the target.

In case of the detection of methylation many times a simultaneous quantification of the amount of variant in the sample is desired. In order to achieve the quantification of a methylated sequence in the presence of an unmethylated or semi-methylated sequence, real time PCR is combined with the use of blockers. Suitable methods are known to those skilled in the art (see for example but not limited to the HM (HeavyMethyl) method US7,229,759; WO 02/072880 or the HQM method PCT/EP2007/006810).

But the use of blockers comprises also disadvantages: Blockers are always directed against a single variant sequence while usually only one variant is of interest. In case a region has to be analyzed comprising two or more variable positions, more than two variable sequences are possible. The additional variants not of interest and not blocked are hence also amplified. These additional amplificates falsify the results of the detection/quantification of the variant of interest. This is for example the case, in the detection of two or more SNPs or in the detection or quantification of two or more positions capable of being methylated. In such case several blocker sequences have to be used in parallel

### DETAILED DESCRIPTION OF ASPECTS OF THE INVENTION

For achieving various technical objects, particular aspects of the invention teach and provide a method for amplification, comprising
- bringing into contact the template to be amplified with a primer and a facilitator,
   - wherein a region of said facilitator hybridizes specifically to a region of said template,
   - wherein a second region of said facilitator hybridizes specifically to a region of said primer,
   - wherein a second region of said primer hybridizes specifically to a second region of the template; and
- extending said primer.

Particular aspects of the invention teach and provide a method for amplification. According to it, three successful hybrization events are necessary in order to allow the elongation of a molecule to start. In one event, a section of a template of interest and a section of a facilitator hybridize onto each other. In another event, a further section of said template and a section of a molecule to be extended hybridize onto each other. And, in a further event, a further section of said facilitator and a further section of said molecule to be extended hybridize to each other. In doing so, the order of said events is irrelevant. The events occur either one after the other or simultaneously. The molecule to be extended is at least in parts an oligonucleotide or oligomer suitable for initiating the extension reaction. According to said aspects of the invention, facilitators and/or primers as specified herein are suitable for realizing the inventive method.

Particular aspects of the invention teach and provide a facilitator, comprising of
• a template hybridizing region; and
• a primer hybridizing region.

Particular aspects of the invention teach and provide a facilitator. A facilitator is a molecule of at least two parts. One part is able to bind to a template and another part is able to bind to a molecule to be extended. The template binding part is an oligonucleotide or oligomer capable in hybridizing to the template. In a preferred embodiment the part binding to a molecule to be extended is also an oligonucleotide or oligomer capable in hybridizing to said molecule. However, and therewith also preferred, is a facilitator which is able to bind to a molecule to be extended by other means such as but not limited to noncovalent interactions, for example between biotin and streptavidin or antigen and antibody, Accordingly, biotin, streptavidin, antigen or antibody are covalently linked to the facilitator. According to a particular preferred facilitator variant, the facilitator is labeled. Preferebly, said facilitator is labeled by means of a dye, fluorescent dye, chemiluminescent dye, quencher, radioactive label, mass label, or other means as known in the art. Preferably, said facilitator is labeled on it's 3' end.

Particular aspects of the invention teach and provide a kit, comprising
• a facilitator according to the invention;
• an enzyme for primer extension, preferably a polymerase and/or a ligase;
• a primer comprising
   - a region hybridizing to the primer-hybridizing region of the facilitator, and
   - a region hybridizing to the template.

Particular aspects of the invention teach and provide a kit. Besides an inventive facilitator, said kit comprises a molecule to be extended or one or more enzymes suitable for catalyzing the amplification reaction. Said molecule to be extended consists of at least two parts. One part of it is an oligonucleotide or oligomer capable in hybridizing onto the template. Another part of it is capable in binding to said fascilitator. Preferably, this part is also a oligonucleotide or oligomer and hybridizes onto the facilitator by hybridization. However, and therewith also preferred, is a molecule to be extended which is able to bind to a facilitator by other noncovalent means such as but not limited to biotin streptavidin interactions or antigen antibody interactions. According to a particular preferred oligonucleotide or oligomer variant hybridizing to the facilitator, the oligonucleotide or oligomer is labeled. Preferebly, said oligonucleotide or oligomer is labeled by means of a dye, fluorescent dye, chemiluminescent dye, quencher, radioactive label, mass label, or other means as known in the art. Preferably, said oligonucleotide or oligomer is labeled on it's 5' end. In a preferred embodiment, an enzyme suitable for catalyzing the amplification reaction is a polymerase, in particular a heatstable polymerase. In another preferred embodiment, enzymes suitable for catalyzing the ampification reaction are a polymerase and a ligase, in particular heatstable polymerase and a heatstable ligase. In a further preferred embodiment, an enzyme suitable for catalyzing the amplification reacition is a ligase, prefereably a heatstable ligase.

Particular aspects of the invention teach the use of the herein taught and provided methods as well as the herein taught facilitators and kits.

### Advantages of Aspects of The Invention.

Particular aspects of the invention are characterized in that they have an enhanced specificity when compared to conventional amplification methods. The specificity of conventional amplification reactions is determined by one hybridization event per molecule to be extended. Said event is the hybridization of the said molecule to the template. Thus, for example, primer extension is characterized by the specificity of one hybridization event because only one molecule is used for extension. Conventional PCR or LCR are characterized by the specificity of two hybridization events because two molecules are used for amplification. In addition the specificity of said amplification reactions can be increased by the use of a blocker i.e. the absence of a hybrization event. Elongation or amplification only occurs in case the blocker does not bind to the template. From this it follows that the maximum specificity of a conventional amplification reaction is the presence of two hybridization events and the absence of a third one.

In contrast to this, the invention provides means and methods which increase the number of hybrization events and thus the specificity of amplification reactions. According to the invention, a molecule is only extended wherein three hybrization events take place: (1) the binding of the molecule to be extended and the template onto each other; (2) the binding of the molecule to be extended and the facilitator onto each other; and (3) the binding of the facilitator and the template onto each other. Thus primer extension using an inventive facilitator is characterized by three hybridization events or in case of the use of a blocker by three hybridization events and the absence of one hybridization event. Correspondingly, PCR or LCR using one inventive facilitator are characterized by four hybridization events or four hybridization events and the absence of one hybridization event. The specificity is further increased by the use of two inventive facilitators, one for each molecule to be extended. Thus, PCR or LCR are characterized by six hybridization events or by six hybridization events and the absence of one hybridization event in case an additional blocker is used.

The use of a facilitator has a fundamental advantage in comparison to the use of a blocker, that provides sofar the highest possible specificity. The mode of operation of a blocker is to hybridize to a undesired sequence and to subpress its amplification. Concurrently, the desired sequence is amplified. However, a blocker has the disadvantage that it can only be specific for one sequence. In the case wherein a sequence of interest has to be amplified in the presence of several undesired sequences, a blocker can only prohibit the amplification of one undesired sequence. The use of two or more blockers is many times not possible without effecting the efficiency of the amplification of the wanted sequence as well. Situations wherein one sequence has to be selectively amplified in the presence of several unwanted sequences are for example but not limited to (i) the amplification or detection of a SNP or mutation allel in the presence of three or four SNP or mutation allels; (ii) the amplification, detection or quantification of the methylation of one CpG position after bisulfite treatment of genomic DNA, wherein said position is either holo-methylated, hemi-methylated or holo-unmethylated; or (iii) the amplification, detection or quantification of a methylation pattern after bisulfite treatment of genomic DNA, in the presence of different methylation patterns of the same CpG positions.

In addition to the increase of specificity, the use of facilitators provides further advantages. New multiplex applications become possible by means of the inventive facilitator. The use of facilitators allows simple and fast multiplex reactions wherein a subclass of amplificates is amplified. For this, for example, the template binding region of the primer is designed so that it is specific for a repeating sequence of interest. Possible sequences are for example but not limited to recognition sites of enzymes, consensus sequences of DNA-binding or RNA-binding proteins such as but not limited to transcription factors, repeats or sequences capable in being methylated such as but not limited to CpG positions. Subclasses of such sequences of interest are amplified by subjecting in addition to said primer a multiplicity of facilitators, wherein each facilitator is specific for a specific site. By use of different primers that are specific for other sequences, it is also possible to amplify different subclasses simultaneously. If the case may be, the amplified subclasses can be easily further amplified in a second amplification reaction by means of primers that are specific for the facilitator-binding regions of the first used primers.

Of course it is also possible to amplify such subclasses by designing the template binding region of the facilitator so that it is specific for said sequences of interest and by a multiplicity of site specific primers. Here again, it is possible to amplify different subclasses by means of different facilitators specific for sequences of interest. Subsequent amplification may be easily performed by means of primers that are specific for the primer-binding regions of the first used facilitators.

Moreover, the complexity of inventive multiplex reactions can even be increased by applying different primers and facilitators specific for different said sequences of interest.

Sofar nothing comparable is possible by means of conventional amplification methods.

In particular the use of a facilitator provides several advantages for the analysis of methylation. Methylated cytosines have the same hybridization behaviour as unmethylated cytosines. In order to distinguish between methylated and unmethylated cytosines, genomic DNA is many times subjected to a conversion reaction wherein unmethylated cytosine is converted to uracil while methylated cytosine remains unchanged. The absence or presence of methylation of a CpG position is determined or quantified by detecting or quantifying the sequence variants at this position after conversion. However, the said conversion has a big disadvantage: the complexity of the DNA is dramatically reduced. During conversion every unmethylated cytosine, that is at least every cytosine outside of a CpG position, is converted to uracil. Thus the four base alphabet of normal DNA is reduced to a three base alpabet of converted DNA over long stretches of DNA. This reduction of complexitiy leads to several disadvantages of methods for methylation analysis.

A well known method for methylation analysis is methylation-specific PCR (MSP). According to the MSP method, genomic DNA is converted by means of bisulfite and amplified by means of methylation-specific primers. In order to ensure methylation-specificity, MSP primers have to cover a sufficient amount of CpG positions. Usually they have to comprise three or more co-methylated CpG positions. In addition, they have to cover three or more positions of cytosine outside of CpG position (unmethylated cytosine) in order to ensure a sufficient efficient discrimination between successfully converted DNA and unconverted DNA. Because the at least six necessary positions (three or more CpG positons and three or more cytosine positions outside of CpG positions) are many times not located next to each other, MSP primers are usually comparably long. On the other side, this increased length results in a higher likelihood of unspecific binding to converted DNA and the primers themselves (so-called primer dimers are formed), in particular because of the reduced complexity of the converted DNA and thus also of the primers.

However, this disadvantages can easily be overcomed by the use of inventive facilitators. According to the invention, both primer as well as facilitator bind to the template DNA. Thus primer and facilitator together have to fullfil the both specified requirements made for MSP primers. Accordingly, a primer may be very short (in extreme cases up to 6, 5, or 4 bases) and so ensuring a high specificity. The corresponding facilitator on the other hand comprises the missing CpG positions and/or the missing cytosine positions outside of CpG positions. In an extreme case, at least three CpG positions and at least three cytosine positions outside of CpG positions are coverd by the facilitator. Of course, also the facilitator might be very short (in extreme cases up to 6, 5, or 4 bases) and so ensuring a high specificity. Then, the primer has to be sufficiently long and to cover the missing CpG positions and/or the missing cytosine positions outside of CpG positions.

Another well known method for methylation analysis is HeavyMethyl (HM) method. According to the HM method, genomic DNA is bisulfite converted and amplified by means of methylation-unspecific primers and a methylation-specific blocker. Said blocker comprises usually three or more CpG positions. The HM method has the disadvantage that only one blocker is applicable that is specific for one target sequence. Many times the target sequence of the blocker is the sequence representing the situation wherein all CpG sites covered by the blocker are unmethylated. Thus sequences representing partial methylation, incompletely converted DNA, or DNA with mutations are amplified in the presence of a blocker as well as the sequence of interest representing the situation wherein all CpG positions covered by the blocker are methylated. Thus, the HM method leads to false-positive results. However, in order to avoid such false-positive results, it is not possible to apply additional blockers. Such additional blockers would affect the amplification of the sequence of interest. Thus, less sequences of interest would be detected than actually present in the sample.

However, these problems can be easily prevented by replacing the blocker by a facilitator. Said facilitator allows the amplification of interest to which it binds but has no effect on sequences representing partial methylation, or does not amplify DNA with mutations. Accordingly, falsified results are nearly completly avoided.

Correspondingly, the inventive method of amplification using the inventive facilitator is advantageous in comparison to other methods of amplification wherein an oligonucleotide or oligomer is used that has at least in parts a blocking function. Such methods are for example but not limited to methods using Headloop primers (WO03/072810; Rand et al., Nucleic Acids Research, 33, e127).

### Method of Aspects of the Invention.

Aspects of the present invention relate to a method for amplification. The method of the invention comprises the formation of a complex consisting of the template, the molecule to be extended such as but not limited to a primer, and an inventive facilitator. In this complex, a region of the facilitator is hybridized specifically to a region of the template, a further region of the facilitator is hybridized specifically to a region of the molecule to be extended, and a further region of the molecule to be extended is hybridized specifically to a further region of the template. Subsequent to the formation of the complex the molecule to be extended is extended.

According to aspects of the present invention, a section of a template of interest hybridizes to a section of a facilitator. In addition, a further section of said template hybridizes to a section of a primer, wherein said further section of said template is located upstream relative to the section hybridizing to the facilitator on the template. Thus the 5' end of the facilitator binding section is located upstream of the 3' end of the primer binding section on the template. In a preferred embodiment, the 5' end of the facilitator binding section is located immediately adjacent to the 3' end of the primer binding section on the template.Furthermore, a further section of said facilitator hybridizes to a further section of said primer, wherein said further section of said facilitator is located downstream of the template binding section on the facilitator, and wherein said further section of the primer is located upstream of the facilitator binding section on the primer. Thus, on the facilitator, the 5' end of the primer-binding section is located in front of the 3' end of the template-binding section, and on the primer, the 3' end of the facilitator-binding section is located in front of the 5' end of the template-binding section. According to aspects of the invention, the order of hybridization is irrelevant. According to aspects of the invention, the hybridizations occur simultaneously or one after the other. According to aspects of the invention, the extension of the primer i.e. the amplification starts only wherein primer, facilitator and template are sequence-specifically hybridized onto each other.

Particular aspects of the present invention relate to a method for amplification, comprising
(a) bringing into contact the template to be amplified with a primer and a facilitator,
   - wherein a region of said facilitator hybridizes specifically to a region of said template,
   - wherein a second region of said facilitator hybridizes specifically to a region of said primer,
   - wherein a second region of said primer hybridizes specifically to a second region of said template; and
(b) extending said primer.

According to an embodiment, the template is a nucleic acid. Said nucleic acid is either naturally occuring nucleic acid, a nucleic acid derived from a naturally occuring nucleic acid, an alterated nucleic acid, or an artificial nucleic acid. According to a preferred embodiment, the template is DNA, RNA, PNA or a derivate thereof. Preferably the template is genomic DNA. According to a particular preferred embodiment, the template is genomic DNA that has been treated so that unmethylated cytosines are converted to uracil or to another base which differs in its base pairing behaviour to cytosine, while methylated cytosines remain unchanged. Preferably, such a treatment is a chemical treatment or an enzymatic treatment. Suitable treatments are known to those skilled in the art. Particularly preferred are genomic DNA treated with bisulfite and genomic DNA treated with cytosine deaminases.

According to a preferred embodiment, DNA is converted by a treatment with a bisulfite. Suitable bisulfite reagents or solutions are known to those skilled in the art. The bisulfite conversion may take place both in solution as well as also on DNA bound to a solid phase. Preferably sodium disulfite (= sodium bisulfite/sodium metabisulfite) is used, since it is more soluble in water than sodium sulfite. The disulfite salt disproportionates in aqueous solution to the hydrogen sulfite anions necessary for the cytosine conversion. When bisulfite concentration is discussed below, this refers to the concentration of hydrogen sulfite and sulfite anions in the reaction solution. For the method according to the invention, concentration ranges of 0.1 to 6 mol/I are possible. Particularly preferred is a concentration range of 1 to 6 mol/l, and most particularly preferred, 2-4 mol/l.

According to a preferred embodiment, a bisulfite treatment is essentially carried out as described in WO05/038051 or in WO 2006/113770. According to this, in one embodiment DNA is reacted with a bisulfite reagent, characterized in that said reaction is carried out in the presence of dioxane or dioxane derivatives or in the presence of n-alkylene glycol compounds, particularly their dialkyl ethers, and especially diethylene glycol dimethyl ether (DME). Preferably, the denaturing solvents are used in concentrations between 1 % and 35 % (v/v). In a preferred embodiment, the n-alkylene glycol compounds, in particular DME are preferably used in concentrations between 1-35% (vol/vol), preferably between 5 and 25%, and most preferably 10% DME.

According to a preferred embodiment, the treatment with a bisulfite comprises a radical scavenger. Preferably the radical scavenger is chromane derivatives, e.g., 6-hydroxy-2,5,7,8,-tetramethylchromane 2-carboxylic acid (also known as: Trolox-C™) or derivative thereof. In a preferred embodiment the concentration for 6-hydroxy-2,5,7,8,-tetramethylchromane 2-carboxylic acid is between 35 and 50 mmol/l. Preferably the radical scavenger is gallic acid (3,4,5- trihydroxy benzoic acid) or a derivative thereof. In a preferred embodiment the concentration for gallic acid and gallic acid derivatives is between 50 and 60 mmol/l.

According to a preferred embodiment, the treatment with a bisulfite comprises a radical scavenger, in particular 6-hydroxy-2,5,7,8,-tetramethylchromane 2-carboxylic acid or gallic acid, and an organic solvent, in particular DME.

Further scavengers usable according to the invention are listed in the patent application WO 01/98528.

The bisulfite conversion can be conducted in a wide temperature range from 0 to 95 °C. However, as at higher temperatures the rates of both the conversion and decomposition of the DNA increase, in a preferred embodiment the reaction temperature lies between 0-80°C, preferably between 30-80°C. Particularly preferred is a range between 50-70 °C; most particularly preferred between 57-65°C. The optimal reaction time of the bisulfite treatment depends on the reaction temperature. The reaction time normally amounts to between 1 and 18 hours (see: Grunau et al. 2001, Nucleic Acids Res. 2001, 29(13):E65-5). The reaction time is ordinarily 4-6 hours for a reaction temperature of 60°C.

In a particularly preferred embodiment of the method according to the invention, the bisulfite conversion is conducted at mild reaction temperatures, wherein the reaction temperature is then increased for a short time at least once during the course of the conversion. In this way, the effectiveness of the bisulfite conversion can be surprisingly clearly be increased. The temperature increases of short duration are named "thermospikes". The "standard" reaction temperature outside the thermospikes is denoted as the basic reaction temperature. The basic reaction temperature amounts to between 0 and 80°C, preferably between 30-80°C, more preferably between 50-70 °C, most preferably between 57-65°C.

The reaction temperature during a thermospike is increased to over 85 °C by at least one thermospike. The optimal number of thermospikes is a function of the basic reaction temperature. The higher the optimal number of thermospikes is, the lower is the basic reaction temperature. Preferably, at least one thermospike is necessary in each case. And, on the other hand, in principle, any number of thermospikes is conceivable. Of course, it must be considered that with a large number of temperature increases, the decomposition rate of the DNA also increases, and an optimal conversion is no longer assured. The preferred number of thermospikes is thus between 1 and 10 thermospikes each time, depending on the basic reaction temperature. A number of two to 5 thermospikes is thus particularly preferred. The thermospikes increase the reaction temperature preferably to 85 to 100°C, particularly preferably to 90-100°C, and most preferably to 94°C-100°C.

The duration in time of the thermospikes also depends on the volume of the reaction batch. It must be assured that the temperature is increased uniformly throughout the total reaction solution. For a 20 µl reaction batch when using a thermocycler a duration between 15 seconds and 1.5 minutes, especially a duration between 20 and 50 seconds is preferred. In a particular preferred embodiment the duration is 30 seconds. Operating on a volume of 100 µl the preferred range lies between 30 seconds and 5 minutes, especially between 1 and 3 minutes. Particularly preferred are 1.5-3 minutes. For a volume of 600 µl, a duration of 1 to 6 minutes, is preferred, especially between 2 and 4 minutes. Particularly preferred is a duration of 3 minutes. A person skilled in the art will easily be able to determine suitable durations of thermospikes in relation to a variety of reaction volumes. The above-described use of thermospikes leads to a significantly better conversion rates in the bisulfite conversion reaction, even when the above-described denaturing solvents are not utilized.

According to the invention, the said treatment of DNA with bisulfite is particularly preferred because it has several important advantages in comparison to other known methods or kits of the state of the art. These advantages are: i) higher yield of converted DNA; ii) a nearly complete conversion of unmethylated cytosine while methylated cytosine remain unchanged; and iii) almost no further fragmentation of DNA. These advantages are based in milder reaction conditions because of i) a thermal denaturation of DNA; ii) a comparably lower bisulfite concentration; iii) a slightly more alkaline pH; and iv) the use of a more efficient and more effective radical scavengers.

In a preferred embodiment, the method of the invention is a method, wherein treating DNA with a bisulfite reagent comprises:
mixing of about 10 to about 250 µl of a solution comprising DNA with about 45 to about 750 µl of bisulfite solution, the bisulfite solution having a pH in the range of about 5.45 to about 5.50 comprising about 4.83 to about 4.93 mol/I hydrogensulfite;
adding about 5 to about 500 µl of an organic radical scavenger solution, the organic radical scavenger solution comprising an organic solvent and about 10 to about 750 mmol/I of 6-hydroxy-2,5,7,8-tetramethyl-chroman-2-carboxylic acid; and
applying a temperature protocol for about 2 to about 18 h, wherein the reaction is conducted in a temperature range of about 0 to about 80°C with about 2 to about 5 additional temperature increases, in each case for about 0.5 to about 10 min, to a temperature of about 85 to about 100°C including an initial temperature increase to a temperature of about 85 to about 100°C.

According to a preferred embodiment, the treatment comprising the use of a bisulfite reagent comprises further:
mixing of about 10 to about 250 µl of a solution comprising DNA with about 45 to about 750 µl of bisulfite solution, the bisulfite solution having a pH in the range of about 5.45 to about 5.50 comprising about 4.83 to about 4.93 mol/I hydrogensulfite;
adding about 5 to about 500 µl of an organic radical scavenger solution, the organic radical scavenger solution comprising an organic solvent and about 10 to about 750 mmol/I of 6-hydroxy-2,5,7,8-tetramethyl-chroman-2-carboxylic acid; and
applying a temperature protocol for about 2 to about 18 h, wherein the reaction is conducted in a temperature range of about 0 to about 80°C with about 2 to about 5 additional temperature increases, in each case for about 0.5 to about 10 min, to a temperature of about 85 to about 100°C including an initial temperature increase to a temperature of about 85 to about 100°C.

In a particular preferred embodiment, the method of the invention is a method wherein treating DNA with a bisulfite reagent comprises:
mixing of about 50 to about 150 µl of a solution containing the DNA with about 95 to about 285 µl of the bisulfite solution;
adding about 15 to about 45 µl of DME solution, the DME solution comprising about 500 mmol/I of 6-hydroxy-2,5,7,8-tetramethyl-chroman-2-carboxylic acid dissolved in diethyleneglycoldimethylether; and
applying a temperature protocol for about 3 to about 16 h, wherein the reaction is conducted in a temperature range of about 57 to about 65°C with about 2 to about 5 additional temperature increases, in each case for about 3 to about 5 min, to a temperature of about 94 to about 100°C including an initial temperature increase to a temperature of about 94 to about 100°C.

According to a particular preferred embodiment, the bisulfite treatment of DNA comprises:
mixing of about 50 to about 150 µl of a solution containing the DNA with about 95 to about 285 µl of the bisulfite solution;
adding about 15 to about 45 µl of DME solution, the DME solution comprising about 500 mmol/I of 6-hydroxy-2,5,7,8-tetramethyl-chroman-2-carboxylic acid dissolved in diethyleneglycoldimethylether; and
applying a temperature protocol for about 3 to about 16 h, wherein the reaction is conducted in a temperature range of about 57 to about 65°C with about 2 to about 5 additional temperature increases, in each case for about 3 to about 5 min, to a temperature of about 94 to about 100°C including an initial temperature increase to a temperature of about 94 to about 100°C.

The said treatment with a bisulfite leads to a sulfonation, a deamination, or both of unmethylated cytosine. Deamination is a spontaneous process in an aqueous solution and leads to sulfonated uracil (uracil sulfonate) comprising DNA. No desulfonation occurs yet.

According to a preferred embodiment, uracil sulfonate is desulfonated to uracil by heating.

According to a preferred embodiment, the DNA after bisulfite treatment and if the case may be after purification is directly subjected to amplification. Suitable methods for purification are known to those skilled in the art. Preferably such a method for purification comprises at leaslt one selected of the group consisting of size exclusion, ultrafiltration, Microcon filter device (Millipore Inc.), filter device, ethanol, propanol, silica surface, silica membrane, magnetic particle, polystyrol particle, positively charged surface, and positively charged membrane, charged membrane, charged surface, charged switch membrane, charged switched surface, column of the ZR DNA Clean & Concentrator-5 Kit (Zymo Research Corporation), column of the Wizard Genomic DNA Purification Kit (Promega U.S.), column of the QIAamp DNA Micro Kit (Quiagen, Inc.), a component of the MagNA Pure Compact Nucleic Acid Isolation Kit (I) Large Volume (Roche Diagnostics GmbH), a component of the OIAamp UltraSens Virus Kit (Quiagen, Inc.), a component of the RTP DNA/RNA Virus Supersense Kit (Invitek Gesellschaft für Biotechnik & Biodesign mbH), a component of the chemagic Viral DNA/RNA Kit special (Chemagen Biopolymer-Technologie Aktiengesellschaft), a component of the chemagic DNA Blood Kit special(Chemagen Biopolymer-Technologie Aktiengesellschaft), a component of the High Pure Viral Nucleic Acid Kit (Roche Diagnostics GmbH), a component of the Puregene DNA Isolation Kit (Gentra Systems, Inc.), a component of the MasterPure™ Complete DNA and RNA Purification Kit (Epicentre Technologies), or a component of the NucliSens^{®} Isolation Kit (bioMérieux SA). In particular preferred, is the purification by Microcon^{™} columns (Millipore Inc.) and the purification by means of SiMAG/FK-Silanol beads (Chemicell GmbH, Germany, Article Number 1101-01 or 1101-05) or beads of the chemagic Viral DNA/RNA Kit special in a previous step (Chemagen Biopolymer-Technologie Aktiengesellschaft; Article Number 1002).

According to a preferred embodiment, uracil sulfonate becomes desulfonated in the initial heating step. If the case may be, the initial heating step lasts for 5 min, 10 min, 15 min, 20 min, 30 min, 45 min or 60 min in order to allow a complete desulfonation of the uracil sulfonate containing DNA. Preferably, the temperature of the initial heating step lies in the range of 80-100°C and most preferably in the range of 90-95°C. The desulfonation by heating treatment is further characterized in that it is performed in low alkaline conditions, preferably in the range of pH 7.5-10, most preferably in the range of pH 8.0-9.0.

According to a preferred embodiment, the treated DNA is cleared from contaminating DNA. For this, the treated DNA comprising sulfonated uracil is brought into contact and incubated with an enzyme which specifically degrades non-sulfonated uracil containing nucleic acids. Such an enzyme is for example but not limited to Uracil-DNA-Glycosylase (UNG; WO 2006/040187).

In a particular preferred embodiment for providing a decontaminated template DNA for polymerase based amplification reactions, the sulfonated and/or deaminated template DNA are mixed with an UNG activity and components required for a polymerase mediated amplification reaction or an amplification based detection assay. After degradation of non-sulfonated uracil containing nucleic acids by use of UNG, the UNG activity is terminated and the template DNA is desulfonated by increased temperature. Subsequently the template DNA is ready to be amplified.

Preferably, degradation, termination, desulfonation and amplification occur in a single tube during a polymerase based amplification reaction and/or an amplification based assay. Preferably such an amplification is performed in the presence of dUTP instead of dTTP.

Preferably, sulfonated and partially or completely deaminated DNA after bisulfite treatment is subjected directly to a polymerase based amplification reaction and/or an amplification based assay without any prior desulfonation. The desulfonation occurs during the initial temperature increase of the amplification reaction.

According to a preferred embodiment, uracil sulfonate is desulfonated to uracil by treatment with an alkaline reagent or solution.

In a preferred embodiment, the desulfonation of bisulfite treated DNA comprises the use of at least one selected from the group consisting of size exclusion, ultrafiltration, Microcon filter device (Millipore Inc.), filter device, ethanol, propanol, silica surface, silica membrane, magnetic particle, polystyrol particle, positively charged surface, and positively charged membrane, charged membrane, charged surface, charged switch membrane, charged switched surface, column of the ZR DNA Clean & Concentrator-5 Kit (Zymo Research Corporation), column of the Wizard Genomic DNA Purification Kit (Promega U.S.), column of the QIAamp DNA Micro Kit (Quiagen, Inc.), a component of the MagNA Pure Compact Nucleic Acid Isolation Kit (I) Large Volume (Roche Diagnostics GmbH), a component of the QIAamp UltraSens Virus Kit (Quiagen, Inc.), a component of the RTP DNA/RNA Virus Supersense Kit (Invitek Gesellschaft für Biotechnik & Biodesign mbH), a component of the chemagic Viral DNA/RNA Kit special (Chemagen Biopolymer-Technologie Aktiengesellschaft), a component of the chemagic DNA Blood Kit special(Chemagen Biopolymer-Technologie Aktiengesellschaft), a component of the High Pure Viral Nucleic Acid Kit (Roche Diagnostics GmbH), a component of the Puregene DNA Isolation Kit (Gentra Systems, Inc.), a component of the MasterPure™ Complete DNA and RNA Purification Kit (Epicentre Technologies), or a component of the NucliSens^{®} Isolation Kit (bioMérieux SA). In particular preferred embodiment, desulfonation is performed by means of Microcon^{™} columns (Millipore Inc.). The said columns are particularly preferred because they show the highest yield of recovery of bisulfite treated DNA when applied to desulfonation. In additon they allow a recovery of small bisulfite treated fragments as well as large bisulfite treated fragments as present in a sample after bisulfite treatment. A person skilled in the art knows to select other suitable devices or kits in considering the above specifications and named kits.

Particularly preferred is a desulfonation of bisulfite treated DNA comprising:
adding of about 50 to about 1000 µl of water to the sample after the bisulfite reaction;
applying the mixture onto a Microcon filter device subsequently centrifuging at about 10,000 to about 18,000 x g for about 10 to about 30 min;
washing with about 100 to about 800 µl of about 0.2 mol/I sodium hydroxide, and subsequent centrifuging at about 10,000 to about 18,000 x g for about 6 to about 25 min;
applying of about 100 to about 800 µl of about 0.1 mol/l sodium hydroxide, and subsequent centrifuging at about 10,000 to about 18,000 x g for about 6 to about 25 min;
applying, in 1 to about 8 repetitions, the following: applying of about 100 to about 400 µl water or TE buffer and subsequent centrifuging at about 10,000 to about 18,000 x g for about 6 to about 25 min; and
eluting by application of about 25 to about 200 µl TE buffer preheated to about 15 to about 65°C, incubation for about 1 to about 30 min at a temperature of about 15 to about 65°C, and subsequent inversion of the Microcon filter device and centrifugation at about 500 to about 5,000 x g for about 0.5 to about 30 min.

Particularly preferred is a desulfonation of bisulfite treated DNA comprising:
a) adding of 200 µl water to the sample after the bisulfite reaction,
b) applying the mixture onto a Microcon filter device subsequently centrifuging at about 14,000 x g for about 20 min,
c) washing with about 400 µl of about 0.2 mol/I sodium hydroxide, and subsequent centrifuging at about 14,000 x g for about 10 to about 14 min,
d) applying of about 400 µl of about 0.1 mol/I sodium hydroxide, and subsequent centrifuging at about 14,000 x g for about 10 to about 14 min,
e) applying, in 1 to about 4 repetitions, the following: applying of about 400 µl water or TE buffer and subsequent centrifuging at about 14,000 x g for about 12 min; and
f) eluting by application of about 45 to about 70 µl TE buffer preheated to about 50°C, incubation for about 10 min at a temperature of about 50°C, and subsequent inversion of the Microcon filter device and centrifugation at about 1,000 x g for about 7 min.

Particularly preferred is a desulfonation of bisulfite treated DNA that comprises at least one of the following:
in step b, applying the mixture in portions onto the Microcon filter device in step b;
subsequent to step b, applying of about 400 µl TE buffer, the TE buffer pH 8 containing about 10 mmol/I tris-hydroxymethyl-amino-methan and about 0.1 mmol/I EDTA, subsequent centrifuging at about 14,000 x g for about 12 min;
in step c, incubating the about 0.2 mol/I sodium hydroxide for about 10 min at room temperature;
in step d, incubating the about 0.1 mol/l sodium hydroxide for about 10 min at room temperature.

According to a preferred embodiment, instead of a chemical treatment, an enzymatic treatment is also possible for the conversion of unmethylated cytosines while methylated cytosines remain unchanged. Suitable enzymes for such a treatment are for example but not limited to methylation-specific cytidine deaminases as they are specified in DE 103 31 107 B or PCT/EP2004/007052.

Altenatively, and herewith also preferred is a treatment of the nucleic acid to be amplified by one or more restriction enzymes. According to the invention, any restriction enzyme is usable. Suitable restriction enzymes are known in the art. Preferably, said restriction enzyme is a methylation-sensitiv restriction enzyme. Preferably, said restriction enzyme is a methylation insensitiv restriction enzyme. Preferably, a combination of methylation-sensitive and methylation-insensitive restriction enzymes is used for said treatment. Said enzymes are either applied simultaneously or one after the other. Preferably, said enzymes are brought into contact with the nucleic acid to be amplified before the facilitator or primer is brought into contact with the nucleic acid to be amplified.

In an embodiment of the invention, the nucleic acid to be amplified is fragmented by chemical, physical and/or mechanical means.

According to a preferred embodiment, the template is selected from the group consisting of genomic DNA, cDNA, chemically treated DNA, bisulfite treated DNA, enzymatically treated DNA, RNA, PNA or derivates thereof.

According to a preferred embodiment, the facilitator does not allow its extension. Preferably, the 3' end of the facilitator is modified for a safe prevention of extension. Suitable modification are known to those skilled in the art. Particulary preferred is a facilitator with any molecule or functional group linked to the 3' position of the 3' terminal nucleotide of the fascilitator. Preferably, said molecule or functional group is linked via a amino link, thiol link, phosphorylation, inverted end (3'-3'linkage), or C3-spacer (propyl spacer). Preferably, said molecule or functional group is a hydrogen atom, an alkyl group, an amino group, a phosphate group, an inverted nucleotide, a C3 spacer (propyl group), or acridine.

According to a preferred embodiment, the 3' end of the facilitator is modified prohibiting the extension of the facilitator.

According to a preferred embodiment, the facilitator consists of two or at least of two sections. One of said two or at least two sections is able to specifically hybridize to the molecule to be amplified. Said section is between 9 to 30 preferably between 12 to 20 nucleotides or monomers long. Particularly preferred, said section is 12, 13, 14, 15, 16, 17, 18, 19, or 20 nucleotides or monomers long.

According to a preferred embodiment, the region of the facilitator being able to hybridize onto the template consists of 9-30, preferably of 12-20 nucleotides.

According to a preferred embodiment, the facilitator consists of two or at least of two sections. One of said two or at least two sections is able to specifically hybridize to the molecule to be extended e.g. the primer. Said section is between 4 to 20 preferably between 6 to 15 nucleotides or monomers long. Particularly preferred, said section is 6, 7, 8, 9, 10, 11, 12, 13, 14, or 15 nucleotides or monomers long.

According to a preferred embodiment, the molecule to be extended e.g. the primer consists of two or at least of two sections. One of said two or at least two sections is able to specifically hybridize to the facilitator. Said section is between 4 to 20 preferably between 6 to 15 nucleotides or monomers long. Particularly preferred, said section is 6, 7, 8, 9, 10, 11, 12, 13, 14, or 15 nucleotides or monomers long.

According to a preferred embodiment, the region of the facilitator being able to hybridize to the primer and the region of the primer be able to hybridize to the facilitator consist each of 4-20, preferably 6-15 nucleotides.

According to a preferred embodiment, the molecule to be extended e.g. the primer consists of two or at least of two sections. One of said two or at least two sections is able to specifically hybridize to the molecule to be amplified. Said section is between 4 to 20 preferably between 6 to 12 nucleotides or monomers long. Particularly preferred, said section is 6, 7, 8, 9, 10, 11, or 12 nucleotides or monomers long.

According to a preferred embodiment, the region of the primer being able to hybridize onto the template consists of 4-20, preferably 6-12 nucleotides.

According to a preferred embodiment, the elongation of the molecule to be extended e.g. the primer is dependent on the binding of the corresponding facilitator to the molecule to be amplified. Wherein the facilitator binds specifically to the molecule to be amplified, the facilitator bound molecule to be extended is elongated by at least one nucleotide or monomer. Wherein the facilitator binds unspecifically to the molecule to be amplified, the facilitator bound molecule to be extended is not elongated by at least one nucleotide or monomer.

According to a preferred embodiment, the primer extension is dependent on the successful hybridization of the facilitator onto the template.

According to a particular preferred embodiment, the facilitator is specific for cytosine methylation. Preferably, the facilitator only binds to the template molecule wherein a defined CpG position of a precurser of the template molecule is methylated. Preferably, the facilitator only binds to the template molecule wherein a defined CpG position of a precurser of the template molecule is unmethylated. Preferably, the facilitator hybridizes to a region of the template that comprises a chemically treated or an enzymatically treated CpG position. Preferably said CpG position is bisulfite treated. Preferably, said CpG position is treated by restriction enzymes, in particular those which digest DNA only in case it is methylated, but in particular also of those which digest DNA in case it is unmethylated. Preferably, the facilitator hybridizes to a region of a template which comprises 1, 2, 3, 4, 5, 6, 7, 8, 9, 10 or more treated CpG positions.

According to a particular preferred embodiment, the primer is specific for cytosine methylation. Preferably, the primer binds only to the template molecule wherein a defined CpG position of a precurser of the template molecule is methylated. Preferably, the primer only binds to the template molecule wherein a defined CpG position of a precurser of the template molecule is unmethylated. Preferably, the primer hybridizes to a region of the template that comprises a chemicaly treated or an enzymatically treated CpG position. Preferably said CpG position is bisulfite treated. Preferably, said CpG position is treated by one or more restriction enzymes, in particular an enzyme which digests DNA only in case it is methylated, but also in particular, an enzymes which digest DNA in case it is unmethylated. Preferably, the primer hybridizes to a region of a template which comprises 1, 2, 3, 4, 5, 6, 7, 8, 9, 10 or more treated CpG positions.

According to a particular preferred embodiment, the facilitator, the primer, or each of the said hybridize to a region of the template that comprises a chemically treated, an enzymatically treated, a restriction enzyme treated or a bisulfite treated CpG position.

According to a particular preferred embodiment, the method of the invention comprises a second primer. Said second primer is specific for amplifying the reverse complementary strand of the template molecule. Said second primer hybridizes onto the elongation product of the first primer. If the case may be, said second primer is only elongated wherein a corresponding second facilitator is also hybridized to the reverse complementary strand. The formation of this second facilitator-primer-template-complex occurs also accoriding to the invention i.e. as it is described above for the first facilitator-primer-template-complex. Of course, and herewith also preferred, is a second primer that is elongated independently of a corresponding facilitator.

According to a particular preferred embodiment, the method of the invention comprises a blocker molecule. Said blocker is applied wherein a template has to be amplified in the presence of a nucleic acid that is similar to the template. For example but not limited to, said similar nucleic acid and said template are allelic variants, SNPs, or mutations. Particularly preferred is the use of blockers in the situation wherein said similar nucleic acid and said template are derivatives of nucleic acids having the same sequence but different methylation. Preferably, said blocker prevents or suppresses the elongation of one of the said two nucleic acids while the other is amplified. Preferably, said blocker prevents or suppresses elongation by prohibiting the complete hybridization of the facilitator onto the template. Preferably, said blocker prevents or suppresses elongation by prohibiting the complete hybridization of the primer onto the template. Preferably, said blocker prevents or suppresses elongation by prohibiting the elongation of the primer.

According to a particular preferred embodiment, the inventive method additionally comprises
(a) a second primer being specific for the reverse complementary sequence of the template;
(b) a blocker either prohibiting the binding of the facilitator, the binding of the primer, the extension of the primer or combinations thereof; or
(c) both. Thereby, said blocker is designed to bind sequences not to be amplified.

According to a particular preferred embodiment, one facilitator and one corresponding primer are applied for elongation. However, the invention also comprises multiplexing embodiments. According to a particularly preferred embodiment for multiplexing, different facilitators and one corresponding primer is applied for elongation. The template-hybridizing region of said facilitators have a variable sequence and hence bind to one or more template sequences, while their primer-hybridizing sequence is identical. For example, but not limited to a pool of different facilitators is designed to hybridize to a multitude of CpG-rich regions within the genome, whereas the template-hybridizing region of the primer is designed to bind to the sequence of one or more defined methylation-sensitive restriction sites.

According to a particularly preferred embodiment for multiplexing, one facilitator and different corresponding primers are applied for elongation. The template-hybridizing region of said primers have a variable sequence and hence bind to one or more template sequences, while their facilitator-hybridizing sequence is identical. For example, but not limited to a pool of primers is designed to hybridize to a multitude of CpG-rich regions within the genome, whereas the correspondent facilitator being specific for said pool is designed to bind to a sequence of one or more defined methylation-sensitive restriction sites. Likewise the primers are designed to bind to defined transcription factor or other protein binding sites.

According to a particularly preferred embodiment for multiplexing, different facilitators and different corresponding primers are applied for elongation. Said facilitators have a variable sequence that allows the binding of the facilitators to multiple template sequences. In addition, the primer-binding sequences of said different facilitators are different. Preferably, the primer-binding sequences of said different facilitators allow the binding of the different facilitators to different subgroups of said different primers.

According to a particularly preferred embodiment for multiplexing, different facilitators and different corresponding primers are applied for elongation. Said primers have a variable sequence that allows the binding of the primers to multiple template sequences. In addition, the facilitator-binding sequences of said different primers are different. Preferably, the facilitator-binding sequences of said different primers allow the binding of the different facilitators to different subgroups of said different facilitators. For example, but not limited to, facilitators are applied to multiplex PCR i.e. multiple pairs consisting of a site specific facilitator and corresponding site specific primer are applied to one amplification reaction.

According to a particular preferred embodiment, different facilitators are applied, the regions of the facilitators hybridizing to the template have a variable sequence, the regions of the different facilitators hybridizing to the primer have the same sequence, and wherein one or more facilitator dependent primers are applied.

According to a particular preferred embodiment, the template-binding regions of different applied primers have a variable sequence. According to a particular preferred embodiment, only parts fo the template-binding regions of different applied primers have a variable sequence. According to a particular preferred embodiment, the template-binding regions of different applied primers varry only in 1, 2, 3, 4, 5, 6, 7, 8 9, or 10 positions. For example, but not limited to, the template binding region of different applied primers consists of a non-variable sequence and additional bases, said non-variable sequence is specific for a recognition site of a restriction enzyme or any other consensus sequence, whereas the additional bases are degenerate.

According to a particular preferred embodiment, the regions of the primers hybridizing to the template have, at least in parts, a variable sequence.

According to a particular preferred embodiment, the template binding region of the primer comprises a consensus sequence, a recognition sequence of an enzyme or protein, a restriction enzyme recognition sequence, or a recognition sequence of a methylation sensitive restriction enzyme.

According to a preferred embodiment, 1, 2,3, 4, or more primers are labeld. A person skilled in the art is aware of suitable labels. Preferably, said label is a fluorescent label, a chemiluminescent label, a mass label, or a radioactive label. Said label are suitable for detecting the amplificates i.e. elongation products. Alternatively and herewith also preferred the, the amplificates i.e. elongation products are detected by intercalating dyes as they are known to those skilled in the art.

Subject matter of the invention is also a facilitator. According to the invention, a facilitator is a molecule which facilitates the elongation of a suitable molecule. However it self cannot be elongated. Preferably, said facilitator consists of at least two parts, two parts, three parts, four parts, or more. Preferably, said facilitator, is at least in parts DNA, PNA (peptide nucleic acid), RNA, GNA (glycerol nucleic acid), TNA (threose nucleic acid), LNA (locked nucleic acid), gripNA, O-MeRNA, a derivative of the said, or combinations thereof. If the case may be said facilitator may also comprise non-nucleic acid parts such as but not limited to biotin, streptavidin, antibody, antigen, quencher, dye for example but not limited fluorophor. Preferably, said non-nucleic acid parts enable or stabilize the binding to the facilitator binding primer.

According to a particular preferred embodiment, the facilitator is DNA, PNA, GNA (glycerol nucleic acid), TNA (threose nucleic acid), LNA (locked nucleic acid), gripNA, O-MeRNA, a derivate thereof, or a combination thereof. According to a preferred embodiment, a facilitator comprises (a) a template-hybridizing region; and (b) a primer-hybridizing region. According to a preferred embodiment, the 3' end of the facilitator is modified prohibiting the extension of the facilitator.

According to a preferred embodiment, the facilitator consists of two or at least of two sections. One of said two or at least two sections is able to specifically hybridize to the molecule to be amplified. Said section is between 9 to 30 preferably between 12 to 20 nucleotides or monomers long. Particularly preferred, said section is 12, 13, 14, 15, 16, 17, 18, 19, or 20 nucleotides or monomers long.

According to a preferred embodiment, the region of the facilitator being able to hybridize onto the template consists of 9-30, preferably of 12-20 nucleotides.

According to a preferred embodiment, the facilitator consists of two or at least of two sections. One of said two or at least two sections is able to specifically hybridize to the molecule to be extended e.g. the primer. Said section is between 4 to 20 preferably between 6 to 15 nucleotides or monomers long. Particularly preferred, said section is 6, 7, 8, 9, 10, 11, 12, 13, 14, or 15 nucleotides or monomers long.

According to a preferred embodiment, the region of the facilitator being able to hybridize to the primer consists of 4-20, preferably 6-15 nucleotides.

According to a particular preferred embodiment, the template-hybridizing region consists of 9-30, preferably 12-20 nucleotides; and the primer hybridizing region consists of 4-20, preferably 6-12 nucleotides.

Preferably, the 3' end of the facilitator is modified for a safe prevention of extension. Suitable modification are known to those skilled in the art. Particulary preferred is a facilitator with any molecule or functional group linked to the 3' position of the 3' terminal nucleotide of the fascilitator. Preferably, said molecule or functional group is linked via a amino link, thiol link, phosphorylation, inverted end (3'-3'linkage), or C3-spacer (propyl spacer). Preferably, said molecule or functional group is a hydrogen atom, an alkyl group, an amino group, a phosphate group, an inverted nucleotide, a C3 spacer (propyl group), or acridine.

According to a particular preferred embodiment, the facilitator is specific for cytosine methylation. Preferably, the facilitator only binds to the template molecule wherein a defined CpG position of a precurser of the template molecule is methylated. Preferably, the facilitator only binds to the template molecule wherein a defined CpG position of a precurser of the template molecule is unmethylated. Preferably, the facilitator hybridizes to a region of the template that comprises a chemicaly treated or an enzymatically treated CpG position. Preferably said CpG position is bisulfite treated. Preferably, said CpG position is treated by restriction enzymes, in particular those which digest DNA only in case it is methylated, but in particular also of those which digest DNA in case it is unmethylated. Preferably, the facilitator hybridizes to a region of a template which comprises 1, 2, 3, 4, 5, 6, 7, 8, 9, 10 or more treated CpG positions.

According to a particular preferred embodiment, the facilitator hybridizes to a region of the template that comprises a chemically treated, an enzymatically treated, a restriction enzyme treated or a bisulfite treated CpG position.

Subject matter of the invention is further a kit. Said kit comprises a container and a facilitator of the invention as described in dteail herein.

According to a particular preferred embodiment, said kit comprises an enzyme for primer extension. Preferably said enzyme is a polymerase and/or a ligase, in particular a heatstable polymerase and/or a heatstable ligase.

According to a particular preferred embodiment, said kit comprises a primer that is only extendable wherein a facilitator and a template are bound to it. Said primer is described in detail above. In brief said primer comprises of a region hybridizing to the primer-hybridizing region of the facilitator, and a region hybridizing to the template.

According to a particular preferred embodiment, said kit comprises one or more of the following: a second primer specific for the reverse complentary sequence of the template; means for amplification such as but not limited to nucleotides, magnesium ions, buffer suitable for polymerase amplification or for ligation; restriction enzymes, in particular one or more methylation sensitive restriction enzyme and/or one or more methylation insensitive restriction enzymes; means for treatment and purification of nucleic acids as specified herein such as bisulfite, deaminase, ultrafiltration device, size exclusion device; means for carry over prevention as herein described such as but not limited to Uracil-DNA-Deglycosylase (UNG); or combinations thereof.

According to a preferred embodiment, the kit of the invention comprises
(a) a container; and
(b) a facilitator of the invention.

According to a preferred embodiment, the kit of the invention comprises additionally
(a) an enzyme for primer extension, preferably a polymerase and/or a ligase;
(b) a primer comprising
   - a region hybridizing to the primer-hybridizing region of the facilitator, and
   - a region hybridizing to the template;
(c) or both.

According to a preferred embodiment, the kit of the invention comprises additionally
(a) a second primer specfic for the reverse complentary sequence of the template;
(b) nucleotides, magnesium ions, buffer suitable for PCR;
(c) bisulfite and/or restriction enzymes, preferably methylation sensitive restriction enzyme and/or methylation insensitive restriction enzyme;
(d) Uracil-DNA-Deglycosylase (UNG); or
(c) combinations thereof.

### Use of a method or a kit of the invention.

Particular aspects of the invention relate to the use of the facilitator of the invention, to the use of the method of the invention or to the use of a kit of the invention.

In particular, the use of a facilitator, a method or kit of the invention is preferred for the analysis of genomic alterations. Such alterations can be any kind of chromosomal or genomic sequence abberation. A person skilled in the art is aware of all such kind of alterations. Preferably, said alterations are deletions, repetitions, inversions, mutations, and single nucleotide polymorphisms (SNPs). In particular said alterations are mutations and SNPs.

In particular, the use of a facilitator, a method or kit of the invention is preferred for the analysis of epigenetic information, in particular the detection and/or quantification of said information. Said epigenetic information is encoded by functional groups as it is well known in the art. Preferably, said epigenetic information is encoded by acetylation or methylation.

Herein, methylation refers to any kind of genomic methylation, however the methylation of cytosine at its C5 atom is particularly preferred.

In particular, the use of a facilitator, a method or kit of the invention is preferred for the analysis of mutation, SNP or methylation.

In particular, the use of a facilitator, a method or kit of the invention is preferred for amplification of sequences. Preferably, said amplification is a amplification wherein only a single sequence, a few sequences (in particular 2, 3, 4, 5, 6, 7, 8, 9, 10) or a multiplicity of sequences (more than 10, more than 20, more than 50, more than 100, more than 500, more than 5000) is amplified. In principle and for this also preferred, the number of sequences is unlimited that are capable to be amplified by means of the inventive facilitator, the inventive method or the inventive kit. Regardless how many sequences are amplified, a single facilitator, a single primer binding to a facilitator, several facilitators, several primers binding to one or more facilitators, a multiplicity of facilitators or a multiplicity of primers binding to a facilitator may be used for amplification. In particular preferred is the use of embodiments as described above.

In particular, the use of a facilitator, a method or kit of the invention is preferred for single plex or multiplex amplification.

All herein cited documents are incorporated by reference to their entirety.

### BRIEF DESCRIPTION OF THE FIGURES.

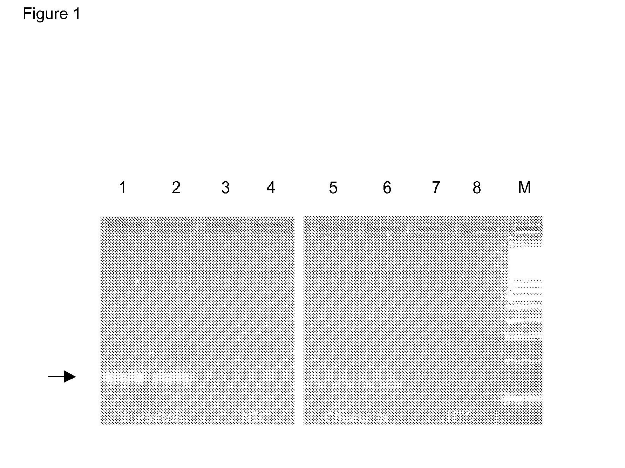

Figure 1 shows the results of an amplification in the presence (lanes 1-4) and in the absence (lanes 5-8) of a facilitator. The used primers and the facilitator were specific for a sequence overlapping with the Vimentin gene (SEQ ID NO: 4; RefSeq DNA: NM_003380). Lanes 1, 2, 5, and 6 show the result of the amplification wherein bisulfite treated methylated genomic DNA was subjected to the reaction ("Chemicon"). Lanes 3, 4, 7, and 8 show the result of the amplification wherein water was subject to the reaction as a control ("NTC"). The arrow indicates the correct product size ("M" size marker). Note, only in the presence of the facilitator the correct product could be amplified from bisulfite treated methylated DNA.

### DEFINITIONS.

In particular aspects, the term "methylation pattern" refers to, but is not limited to, the presence or absence of methylation of one or more nucleotides. Thereby said one or more nucleotides are comprised in a single nucleic acid molecule. Said nucleotides have the ability of being methylated or being non-methylated. Either, all nucleotides of a methylation pattern are methylated, all nucleotides of a methylation pattern are unmethylated, or some of said nucleotides are methylated while the rest of said nucleotides is unmethylated. The term "methylation status" can also be used, but is not limited to, wherein only a single nucleotide is considered. A methylation pattern can be quantified, wherein it is considered over more than one nucleic acid molecule.

In particular aspects, the term "single plex" refers to but is not limited to the amplification of sequences by means of a single facilitator and/or by means of a single primer.

In particular aspects, the term "multiplex" refers to but is not limited to the amplification of sequences by means of two or more facilitators and/or by means of two or more primers.

In particular aspects, the term "hybridization" and/or the term "hybridize" refers to but is not limited to the binding of two or more nucleic acid to each other, wherein said binding is dependent on the sequences of the nucleic acids. In particular preferred is a binding wherein said sequences are, at least in parts, essentially reverse complementary to each other, preferably wherein said sequences are, at least in parts, reverse complementary to each other.

In particular aspects, the term "primer" refers to but is not limited to any oligonucleotide or oligomer that is capable in initiating amplification, in particular that is able to initiate its own extension. Preferably, the term "primer" refers to but is not limited to an oligonucleotide primer of PCR and to a oligonucleotide probe of LCR, respectively.

In particular aspects, the term "holo-methylated" refers to but is not limited to the complete methylation of a CpG position of double stranded DNA. Said CpG position comprises two cytosines capable of being methylated. One cytosine is located in the sense strand while the other cytosine is located in the anti-sense strand.

In particular aspects, the term "holo-unmethylated" refers to but is not limited to the complete unmethylation of a CpG position of double stranded DNA. Said CpG position comprises two cytosines capable of being methylated. One cytosine is located in the sense strand while the other cytosine is located in the anti-sense strand.

In particular aspects, the term "hemi-methylated" refers to but is not limited to the partial methylation of a CpG position of double stranded DNA. Said CpG position comprises two cytosines capable of being methylated. One cytosine is located in the sense strand while the other cytosine is located in the anti-sense strand. Either one of the said cytosines is methylated while the other cytosine is unmethylated.

### EXAMPLE

### Example 1: PCR amplification by means of a facilitator

As indicated in Figure 1, amplification of a sequence of the Vimentin gene (SEQ ID NO: 4; RefSeq DNA: NM_003380) was performed in the presence (lanes 1, 2, 3, and 4) or absence (lanes 5, 6, 7, and 8) of a facilitator. Each reaction was carried out in duplicate. As template bisulfite treated methylated DNA (specified in Figure 1 as "Chemicon") was subjected to the amplification reaction. As control, water was used to the reaction mixture (specified in Figure 1 as "NTC").
forward primer:
   SEQ ID NO: 1 ctctttcctaatgaaattataaaaacaa
reverse primer (indicated as "RP23" in Figure 1):
   SEQ ID NO: 2 gctaagctgca*tgggtgtgggt*
facilitator:
   SEQ ID NO: 3 *cgaacgagggcgcgg*tgcagcttagc-C3

Italicized bases of the reverse primer and of the facilitator refer to bases which are able to hybridize onto the template (target binding region). Underlined bases of the reverse primer refer to bases that are able to hybridize to bases that are underlined in the sequence of the facilitator. (C3 = C3 spacer)

Melting temperature of forward primer: 52°C

Melting temperature of target binding region of reverse primer: 30°

Melting temperature of target binding region of the facilitator : 55°C

The reaction mixtures contained the following:

| lanes of Figure 1 | 1+2 | 3+4 | 5+6 | 7+8 |
|---|---|---|---|---|
| bisulfite treated methylatedDNA | | | | |
| | 10ng/10µl | 0µl | 10ng/10µl | 0µl |
| water as template | 0µl | 10µl | 0µl | 10µl |
| forward primer | 0,5µmol/l | 0,5µmol/l | 0,5µmol/l | 0,5µmol/l |
| reverse primer | 0,5µmol/l | 0,5µmol/l | 0,5µmol/l | 0,5µmol/l |

| Qiagen *HotStarTaq* 10x PCR buffer (comprising 15mmol/l Mag²⁺) | | | | |
|---|---|---|---|---|
| | 1x | 1x | 1x | 1x |
| facilitator | 0,5µmol/l | 0,5µmol/l | 0,5µmol/l | 0,5µmol/l |

| Qiagen *HotStarTaq* DNA polymerase (U = unit) | | | | |
|---|---|---|---|---|
| | 1U | 1U | 1U | 1U |

According to the following scheme, PCR was carried out in a Eppendorff mastercycler cycler: 95°C 15min; 39cycles of 95°C 30s, 58°C 30s, 72°C 45s; afterwards 72°C for 10min and 8°C until analysis in a total volume of 20µl.

Figure 1 shows the results of the amplification. Note, only in the presence of the facilitator, the correct product could be amplified from bisulfite treated methylated DNA.

## Claims

1. A method for amplification, comprising
(a) bringing into contact the template to be amplified with a primer and a facilitator,
- wherein a region of said facilitator hybridizes specifically to a region of said template,
- wherein a second region of said facilitator hybridizes specifically to a region of said primer,
- wherein a second region of said primer hybridizes specifically to a second region of said template; and
(b) extending said primer.

2. A method of claim 1, wherein the template is selected from the group consisting of genomic DNA, cDNA, chemically treated DNA, bisulfite treated DNA, enzymatically treated DNA, RNA, PNA or derivates thereof.

3. A method of claim 1, wherein the 3' end of the facilitator is modified prohibiting the extension of the fascilitor.

4. A method of claim 1, wherein the region of the facilitator being able to hybridize onto the template consists of 9-30, preferably of 12-20 nucleotides.

5. A method of claim 1, wherein the region of the facilitator being able to hybridize to the primer and the region of the primer be able to hybridize to the facilitator consist each of 4-20, preferably 6-15 nucleotides.

6. A method of claim 1, wherein the region of the primer being able to hybridize onto the template consists of 4-20, preferably 6-12 nucleotides.

7. A method of claim 1, wherein the primer extension is dependent on the successful hybridization of the facilitator onto the template.

8. A method of claim 2, wherein the facilitator, the primer, or each of the said hybridize to a region of the template that comprises a chemically treated, an enzymatically treated, a restriction enzyme treated, or a bisulfite treated CpG position.

9. A method of claim 1, additional comprising
(a) a second primer being specific for the reverse complementary sequence of the template;
(b) a blocker either prohibiting the binding of the facilitator, the binding of the primer, the extension of the primer or combinations thereof; or
(c) both.

10. A method of claim 1, wherein different facilitators are applied, the regions of the facilitators hybridizing to the template have a variable sequence, the regions of the different facilitators hybridizing to the primer have the same sequence, and wherein one or more facilitator dependent primers are applied.

11. A method of claim 1, the regions of the primers hybridizing to the template have, at least in parts, a variable sequence.

12. A method of claim 10, the template binding region of the primer comprises a consensus sequence, a recognition sequence of an enzyme or protein, a restriction enzyme recognition sequence, or a recognition sequence of a methylation sensitive restriction enzyme.

13. A facilitator, comprising of
(a) a template hybridizing region; and
(b) a primer hybridizing region.

14. A facilitator of claim 13, wherein
(a) the template hybridizing region consists of 9-30, preferably 12-20 nucleotides; and
(b) the primer hybridizing region consists of 4-20, preferabyl 6-12 nucleotides.

15. A facilitator of claim 13, wherein the facilitator is DNA, PNA, GNA, TNA, LNA, gripNA, O-MeRNA , a derivate thereof, or a combination thereof.

16. A kit comprising
(a) a container; and
(b) a facilitator of claims 13-15.

17. A kit of claim 16, additionally comprising
(a) an enzyme for primer extension, preferably a polymerase and/or a ligase;
(b) a primer comprising
- a region hybridizing to the primer-hybridizing region of the facilitator, and
- a region hybridizing to the template;
(c) or both.

18. A kit of claim 16, additionally comprising
(a) a second primer specfic for the reverse complentary sequence of the template;
(b) nucleotides, magnesium ions, buffer suitable for PCR;
(c) bisulfite and/or restriction enzymes, preferably methylation sensitive restriction enzyme and/or methylation insensitive restriction enzyme;
(d) Uracil-DNA-Deglycosylase (UNG); or
(c) combinations thereof.

19. Use of a method of claim 1-12, a facilitator of claims 13-15 or a kit of claims 16-18 for the analysis of mutation, SNP or methylation.

20. Use of a method of claim 1-12, a facilitator of claims 13-15 or a kit of claims 16-18 for single plex or multiplex amplification.


Sequence listing

<110> Epigenomics AG

<120> FACILITATOR AND METHOD FOR AMPLIFICATION

<160> 3

<210> 1
<211> 28
<212> DNA
<213> Artificial Sequence

<220>
<223> forward primer

<400> 1

ctctttccta atgaaattat aaaaacaa                                          28

<210> 2
<211> 22
<212> DNA
<213> Artificial Sequence

<220>
<223> reverse primer

<400> 2

gctaagctgc atgggtgtgg gt                                                22

<210> 3
<211> 26
<212> DNA
<213> Artificial Sequence

<220>
<223> facilitator

<400> 3

cgaacgaggg cgcggtgcag cttagc                                            26


<210> 4
<211> 1847
<212> DNA
<213> Homo Sapiens

<400> 4

gtccccgcgc cagagacgca gccgcgctcc caccacccac acccaccgcg ccctcgttcg      60
cctcttctcc gggagccagt ccgcgccacc gccgccgccc aggccatcgc caccctccgc     120
agccatgtcc accaggtccg tgtcctcgtc ctcctaccgc aggatgttcg gcggcccggg     180
caccgcgagc cggccgagct ccagccggag ctacgtgact acgtccaccc gcacctacag     240
cctgggcagc gcgctgcgcc ccagcaccag ccgcagcctc tacgcctcgt ccccgggcgg     300
cgtgtatgcc acgcgctcct ctgccgtgcg cctgcggagc agcgtgcccg gggtgcggct     360
cctgcaggac tcggtggact tctcgctggc cgacgccatc aacaccgagt tcaagaacac     420
ccgcaccaac gagaaggtgg agctgcagga gctgaatgac cgcttcgcca actacatcga     480
caaggtgcgc ttcctggagc agcagaataa gatcctgctg gccgagctcg agcagctcaa     540
gggccaaggc aagtcgcgcc tgggggacct ctacgaggag gagatgcggg agctgcgccg     600

gcaggtggac cagctaacca acgacaaagc ccgcgtcgag gtggagcgcg acaacctggc 660
cgaggacatc atgcgcctcc gggagaaatt gcaggaggag atgcttcaga gagaggaagc 720
cgaaaacacc ctgcaatctt tcagacagga tgttgacaat gcgtctctgg cacgtcttga 780
ccttgaacgc aaagtggaat ctttgcaaga agagattgcc tttttgaaga aactccacga 840
agaggaaatc caggagctgc aggctcagat tcaggaacag catgtccaaa tcgatgtgga 900
tgtttccaag cctgacctca cggctgccct gcgtgacgta cgtcagcaat atgaaagtgt 960
ggctgccaag aacctgcagg aggcagaaga atggtacaaa tccaagtttg ctgacctctc 1020
tgaggctgcc aaccggaaca atgacgccct gcgccaggca aagcaggagt ccactgagta 1080
ccggagacag gtgcagtccc tcacctgtga agtggatgcc cttaaaggaa ccaatgagtc 1140
cctggaacgc cagatgcgtg aaatggaaga gaactttgcc gttgaagctg ctaactacca 1200
agacactatt ggccgcctgc aggatgagat tcagaatatg aaggaggaaa tggctcgtca 1260
ccttcgtgaa taccaagacc tgctcaatgt taagatggcc cttgacattg agattgccac 1320
ctacaggaag ctgctggaag gcgaggagag caggatttct ctgcctcttc caaacttttc 1380
ctccctgaac ctgagggaaa ctaatctgga ttcactccct ctggttgata cccactcaaa 1440
aaggacactt ctgattaaga cggttgaaac tagagatgga caggttatca acgaaacttc 1500
tcagcatcac gatgaccttg aataaaaatt gcacacactc agtgcagcaa tatattacca 1560
gcaagaataa aaaagaaatc catatcttaa agaaacagct ttcaagtgcc tttctgcagt 1620
ttttcaggag cgcaagatag atttggaata ggaataagct ctagttctta acaaccgaca 1680
ctcctacaag atttagaaaa aagtttacaa cataatctag tttacagaaa aatcttgtgc 1740
tagaatactt tttaaaaggt attttgaata ccattaaaac tgcttttttt tttccagcaa 1800
gtatccaacc aacttggttc tgcttcaata aatctttgga aaaactc 1847